# EUROPEAN PATENT SPECIFICATION

(11) **EP 1 598 047 B1**
(45) Date of publication and mention of the grant of the patent: **30.11.2011**
(21) Application number: 04012176.6
(22) Date of filing: 22.05.2004
(51) Int. Cl.: A61K 8/49, A61Q 5/10

(54) **Composition for coloring of keratin fibres**
Zusammensetzung zum Färben von Keratinfasern
Composition de teinture de fibres kératiniques

(43) Date of publication of application: 23.11.2005
(73) Proprietor: KPSS-Kao Professional Salon Services GmbH, 64297 Darmstadt (DE)
(72) Inventor: Wood, Jonathan, Dr., 69469 Weinheim (DE); Nöcker, Bernd, Dr., 64372 Ober-Ramstadt (DE); Wilz, Rüdiger, 64319 Pfungstadt (DE)

(56) References cited:
- EP-A- 1 166 752
- WO-A-99/36037
- WO-A-03/022232
- WO-A-03/076518
- US-A- 6 001 135

## Description

The present invention concerns a composition for the dyeing keratin fibers especially human hair on the basis of an oxidation dyestuff precursor system reacting with peroxide and comprising additionally cationic and anionic direct dyes which provides long-lasting, intensive colors either used as such, or which can be used to obtain further shades in combination with additional developing and/or coupling agents.

The developing substances still most frequently used in hair dyeing compositions are 1,4-diaminobenzene (p-phenylenediamine) and 1-methyl-2,5-diaminobenzene (p-toluylenediamine). Although incorporation of these substances largely fulfills the user's color wishes, there are still shades that cannot be completely achieved by the use thereof or which can be intensified. Usually direct dyes of cationic and/or neutral character are used for obtaining further shades. For example, EP 850 636, EP 850 637 and EP 852 135 disclose composition based on oxidative dye precursors and cationic direct dyes. However the colors so obtained are not long last as the cationic dyes are easily washed out by subsequent shampooing and other environmental influences.

WO 03/076518 discloses a process for converting sparingly soluble salts of cationic organic compounds and inorganic acids into more readily soluble salts of organic acids. The cationic compounds disclosed include cationic dyestuff and anionic dyestuffs are suggested for the above process. However, it was not shown and suggested to combine anionic dyes and cationic dyes in order to achieve more durable hair colour.

The invention therefore starts from the problem of creating a hair dyeing composition suited for the preparation of a large number of shades with especially intensive, glossy appearance and being excellently durable.

This problem is solved when such a hair dyeing composition comprises
a- at least one oxidation dyestuff precursor reacting with peroxide, which is selected from pyrazole or the water-soluble salts thereof,
b- at least one acidic direct dye selected from the compounds Acid Red 52, DC Violet 2, DC Red 33, DC Orange 4 and DC Yellow 10, and
c- at least one cationic direct dye selected from the compounds presented with the general formulas III, IV, and V 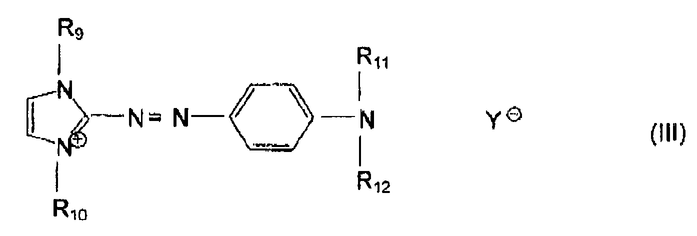 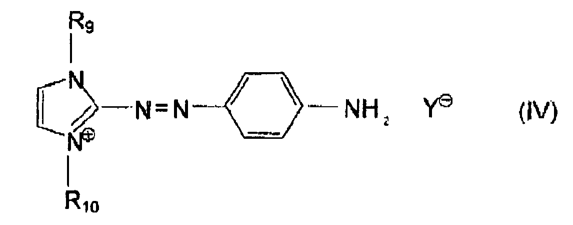 and 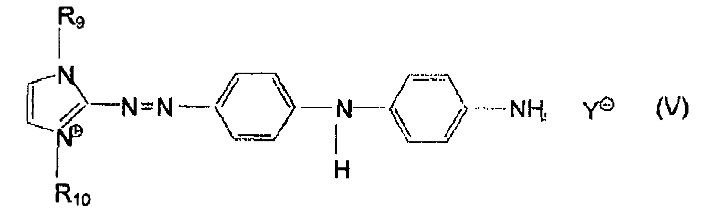
wherein R₉, R₁₀, R₁₁ and R₁₂ stand for hydrogen, a CH₃- or C₂H₅- group, and Y is an anion such as chloride, bromide, methosulfate.

According to the invention, suitable cationic dyestuffs are in principal those available on the market for cosmetic hair colouring applications. For this purpose, special reference is made to the PCT application WO 95/15144 of Ciba-Geigy AG.

Further object of the present invention is a composition for dyeing of keratin fibres, especially human hair, comprising
a- at least one oxidation dye precursor selected from 1-hydroxyethyl-4,5-diaminopyrazole, 3,4-diamino-5-hydroxypyrazole, 3.5-diaminopyrazole, 3.5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts,
b- at least one acidic direct dye selected from Acid Red 52, DC Violet 2, DC Red 33, DC Orange 4 and DC Yellow 10. and
c- at least one cationic direct dye selected from the compounds presenter above with the general formulas III, IV and V.

The most preferred cationic dyes in the colouring compositions of the present invention are the ones according to the formula III, where R₉, R₁₀, R₁₁ and R₁₂ are methyl and Y is chloride, and according to formula IV where R₉, and R₁₀ are methyl and Y is chloride.

The total concentration of the oxidation dyestuff precursors customarily ranges between about 0.05 % and 5 %, preferably 0.1% and 4 %, in particular 0.1% to 3% by weight, calculated to the total hair dyeing composition (excluding the oxidation agent), whereby these figures are always related to the proportion of free base.

According to the invention, coloring composition comprises anionic dyes at a concentration of 0.1 to 7.5%, preferably 0.2 to 5%, more preferably 0.2 to 3% by weight calculated to total composition. Cationic dyestuffs are included into the compositions of the present invention at a concentration of 0.01 to 2.5%, preferably 0.05 to 2% and more preferably 0.05 to 1.5% by weight calculated to total composition.

Interestingly it has been found out that the ratio of acidic dyes to cationic dyes plays an important role for achieving especially resistance to washing and environmental effects. Accordingly, the ratio of cationic dyestuffs to acidic dyestuffs by weight is in the range of 3:1 to 1:10, preferably 2:1 to 1:7 and further more preferably 2:1 to 1:5.

The composition according to the invention preferably comprises at least one coupling substance, which can be selected from resorcinol, 2-methyl, resorcinol, 4-chlororesorcinol, 2-amino-4-chlorophenol, 5-amino-4-methoxy-2-methytphenol. 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxypyridine, 2-dimethyl-amino-5-aminopyridine, 2,6-diaminopyridine, 1,3-diaminobenzene, 1-amino-3-(2'-hy-droxyethylamino)benzene, 1-amino-3-[bis(2'-hydroxy-amino]benzene, α-naphthol, 4,6-dichlororesorcinol, 1,3-diaminotoluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof. However, this shall not exclude the addition of further developing and coupling substances.

The weight proportion of the named developing substances to the additional developing and coupling substances ranges between about 1 : 8 to 8 : 1, preferably about 1 : 5 to 5 : 1, in particular 1 : 2 to 2 : 1. In the hair dyeing compositions according to the invention, the coupling substance(s) as reaction partners of the developing substance(s) are present in approximately the same molecular proportions as the developing substances, i.e. in amounts from 0.01 % to 5.0 %, preferably 0.05 % to 4 %, in particular 0.1 % to 3 % by weight, calculated to the total composition (excluding the oxidizing agent), whereby these figures are always related to the proportion of free base.

After oxidation with peroxide, use of these compositions on the basis of a customary carrier provides very expressive, intensive, long-lasting hair colorations, which can be varied to achieve further shades by the addition of the respective further developing and coupling substances.

It is also possible to incorporate additional developing substances known **per se**. Suitable are tetraaminopyrimidines are in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hy-droxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diaminophenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-(3-hydroxyethyl-2,5-diamino-4-methyl benzene, 2-methoxy-p-phenylenediamine, N,N-diethyl-p-phenylenediamine, 1-amino-β-P-methoxyethyl aminobenzene, 1-dimethyl-amino-4-aminobenzene, 1-hydroxy-2,5-diamino-4-methyl benzene, 1-hydroxymethyl-2,5-diaminobenzene, 1.3-dimethyl-2,5-diaminobenzene, 1.4-diamino isopropyl benzene and/or 1-amino-4-β-hydroxypropyl aminobenzene or the water-soluble salts thereof.

Additionally other cationic dyestuffs can as well be used in addition to the cationic dyestuffs mentioned above. Some examples to those are:

Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.

Additionally, the coloring compositions of the present invention may comprise neutral dyes (HC dyes), so called nitro dyes for shading purposes. Concentration of those can typically be in the range from 0.01 to 2.5%, preferably 0.1 to 2% by weight calculated to total composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No:5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

Hair dyeing composition of the present invention preferably comprise an organopolysiloxane wherein at least one silicium atom is linked to an alkylene group having a hetero-atom, in particular a nitrogen atom, with a poly-(N-acyl alkyleneimine) units of the formula 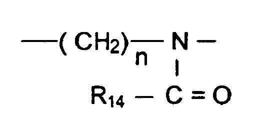 wherein n is a number from 1 to 5 and R₁₄ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 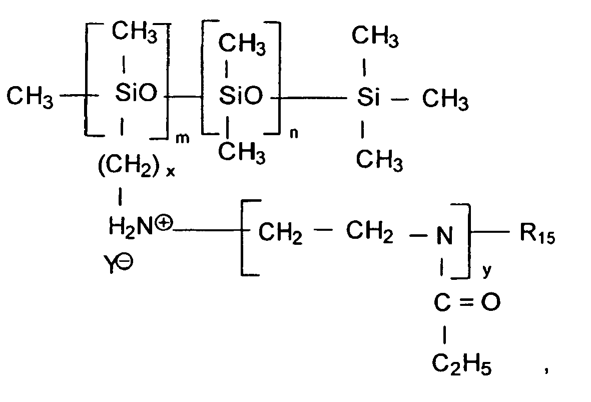 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Another preferred compound in the colouring composition is of ceramide type of compounds according to general formula 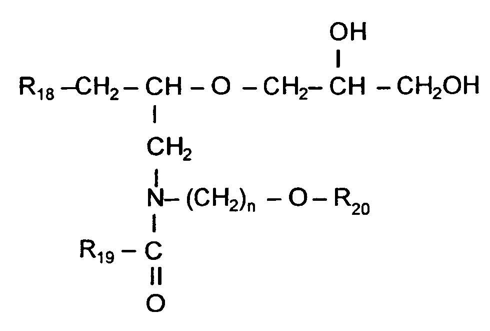 where R₁₈ and R₁₉ are independent from each other alkyl- or. alkenyl group mit 10 to 22 carbon atoms, R₂₀ is methyl, ethyl, n-propyl or isopropyl group and n is a number between 1 to 6, preferably 2 or 3. The concentration of ceramide type of compound in colouring compositions of the present invention can be in the range of 0.01 to 2 and especially 0.01 to 1% by weight calculated to the total composition.

The compositions of the present invention can comprise one or more ubiquinone of the formula. 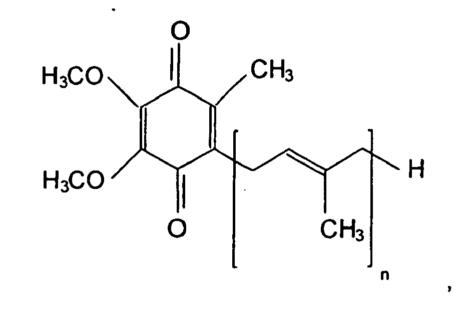 wherein n is a number from 1 to 10. Concentration of ubichinones in the compositions of the present invention can vary between 0.001 %. and 10 %. by weight, calculated to the total composition excluding oxidizing agent.

Coloring composition of the present invention can certainly comprise compounds for accelerating (catalysts) the oxidative dyeing keratin fibres such as iodine salts i.e. potassium or sodium iodide and/or dihydroxy acetone.

The hair dyeing compositions according to the invention can comprise the basic substances and additives customarily found in such compositions, conditioning agents, etc., known as state of the art and described, for example, in the monography of K. Schrader, "Grundlagen und Rezepturen der Kosmetika", 2nd Ed. (Hüthig Buch Verlag, Heidelberg, 1989), pp. 782 to 815. They can be prepared as solutions, creams, gels or also in the form of aerosol products; suitable carrier material compositions are known as state of the art.

For application, the oxidation dyestuff precursor is mixed with an oxidizing agent. The preferred oxidizing agent is hydrogen peroxide, for example in a concentration of 2 to 12 % by weight. However, the use of other peroxides such as urea peroxide and melamine peroxide is also possible.

As an alternative to peroxide oxidation, it is also possible to achieve the oxidation by air, for example, by applying onto the hair a composition comprising an oxidation dyestuff precursor as aerosol foam and leaving to process for about 15 to 20 minutes.

The pH-value of the ready-to-use hair dyeing composition, i.e. after mixing with peroxide, can be between 5 and 12, preferably 6 - 11, more preferably 6.8 to 10.

The following examples are to illustrate the invention without limiting it.

| Carrier | |
|---|---|
| Stearyl alcohol | 8.0 (% by wt.) |
| Coco fatty acid monoethanolamide | 4.5 |
| 1.2-Propanediol mono/distearate | 1.3 |
| Coco fatty alcohol polyglycolether | 4.0 |
| Sodium lauryl sulfate | 1.0 |
| Oleic acid | 2.0 |
| 1.2-Propanediol | 1.5 |
| Na-EDTA | 0.5 |
| Sodium sulfite | 1.0 |
| Protein hydrolyzate | 0.5 |
| Ceramide according to formula where | 0.2 |
| R₁₈ and R₁₉ are C16 and R₂₀ is ethyl | |
| Ascorbic acid | 0.2 |
| Organopolisiloxane according to EP640643 | 0.3 |
| Compound A-1 | |
| Perfume | 0.4 |
| Ubichinone 10 | 0.1 |
| Ammonia, 25% | 1.0 |
| Ammonium chloride | 0.5 |
| Panthenol | 0.8 |
| Water | ad 100.00 |

The dyestuff combinations either oxidative or direct dyes of anionic and cationic characters were incorporated into this carrier, whereby the water content was reduced accordingly.

The colorations were carried out on wool patches, strands of bleached human hair and natural human hair at a color level of 8 by application of a 1:1 mixture of an emulsion comprising the dyestuffs as given in the examples below and a 6 % hydrogen peroxide solution (pH-value of the mixture: 9.8) with 30 minutes processing at room temperature, subsequent rinsing and drying.

The following colorations were obtained:

**Table I: Examples 1 to 5**

| | Example | | | | |
|---|---|---|---|---|---|
| | I | II | III | IV | V |
| | (all values are weight %) | | | | |
| 1-Hydroxyethyl-4,5-diamino-pyrazole sulfate | 1.05 | 1.05 | 1.05 | 1.05 | 1.05 |
| 4-amino-3-hydroxytoluene | 0.54 | 0.54 | 0.54 | 0.54 | 0.54 |
| R₉=R₁₀=R₁₁=R₁₂= methyl | 0.06 | 0.04 | 0.02 | 0.08 | - |
| and Y is chloride | | | | | |
| Acid red 52 | 0.02 | 0.04 | 0.06 | - | 0.08 |

Examples I to III are according to the invention and examples IV and V are for comparative purposes.

The coloring compositions so obtained show excellent dyeing performance.

In order show color fastness (durability) against washing, all colored strands were washed 10 times under usual hair wash conditions with a commercial shampoo composition designed for colored hair of the trade mark Goldwell Definition, and color of the tresses (L, a and b values) were measured before and after shampooing optically with a laboratory equipment and color differences were calculated with the well known equation to obtain ΔE values and color intensity differences (ΔL) were obtained from measured L values. The results are presented in the Table II below.

**Table II: Results of the durability test**

| Example | L1 | L2 | ΔE |
|---|---|---|---|
| I | 35.6 | 37.0 | 7.8 |
| II | 37.0 | 38.9 | 5.8 |
| III | 37.5 | 40.6 | 6.7 |
| IV | 35.3 | 40.5 | 10.5 |
| V | 36.0 | 42.0 | 11.0 |

L1 stands for the intensity of the color measured before the washing test and L2 is the same value measured after washing the strands 10 times with shampoo. ΔE value is obtained from the L, a and b values measured before and after washing. As it is obvious from the table immediately after coloring there is no real difference in the intensity though shade differences were obvious (not shown). However, after washing the strands with shampoo, intensity differences were obvious between the strands colored only with either cationic or anionic dyes (non inventive examples IV and V, respectively) and the strands colored with mixture of anionic and cationic dyes according to the present invention. This is furthermore expressed with ΔE values as the color difference.

## Claims

1. Composition for the dyeing of keratin fibers, especially human hair, **characterized in that** it comprises
a- at least one oxidation dye precursor selected from pyrazole or the water-soluble salts thereof,
b- at least one acidic direct dye, selected from the compounds Acid Red 52, DC Violet 2, DC Red 33, DC Orange 4, and DC Yellow 10 and
c- at least one cationic direct dye selected from the compounds presented with the general formulas III, IV, and V 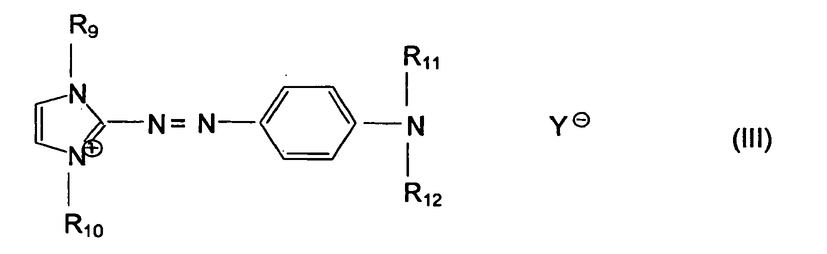 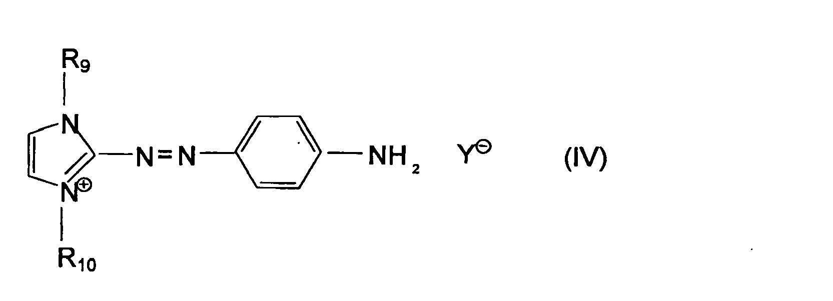 , and 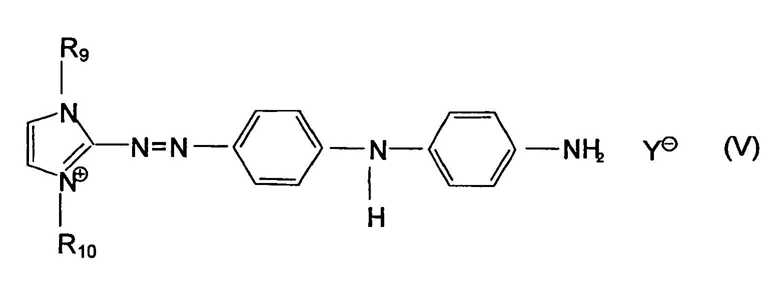 wherein R₉, R₁₀, R₁₁ and R₁₂ stand for hydrogen, a CH₃- or C₂H₅- group,
and Y is an anion such as chloride, bromide, methosulfate.

2. composition for the dyeing of keratin fibers, especially human hair, **characterized in that** it comprises
a- at least one oxidation dye precursor selected from the compounds 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts ,
b- at least one acidic direct dye, selected from the compounds Acid Red 52, DC Violet 2, DC Red 33, DC Orange 4, and DC Yellow 10 and
c- at least one cationic direct dye selected from the compounds presented
with the general formulas III, IV, and V 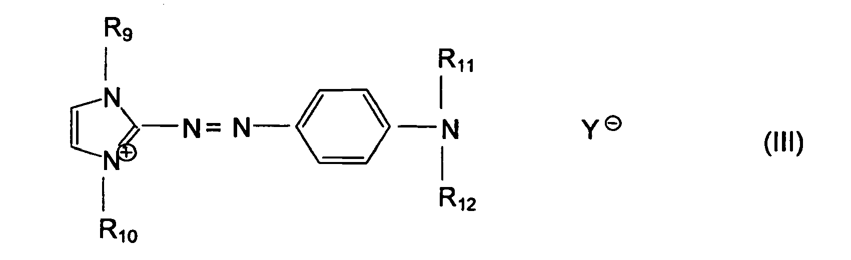 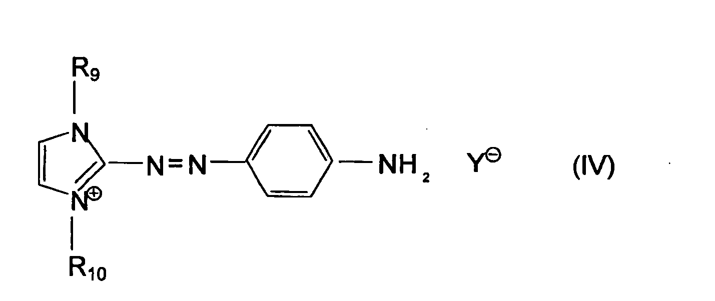 , and 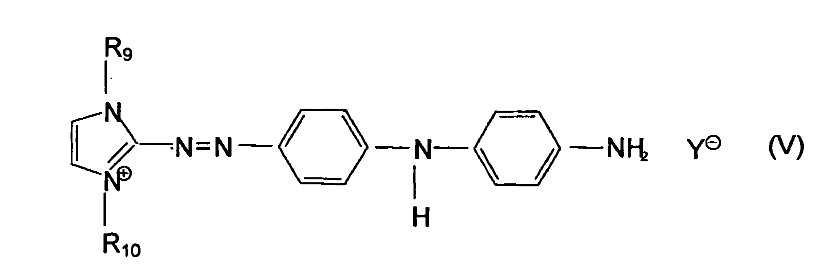 wherein R₉, R₁₀, R₁₁ and R₁₂ stand for hydrogen, a CH₃- or C₂H₅- group, and Y is an anion such as chloride, bromide, methosulfate.

3. Composition according to any of the preceding claims **characterized in that** it comprises as the component c the cationic dyestuff according to formula III 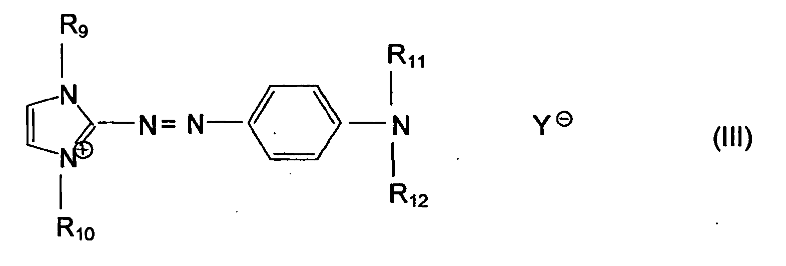 wherein R₉, R₁₀, R₁₁ and R₁₂ are methyl and Y is chloride.

4. Composition according to claims 1 to 3 **characterized in that** it comprises as the component c the cationic dyestuff according to formula IV 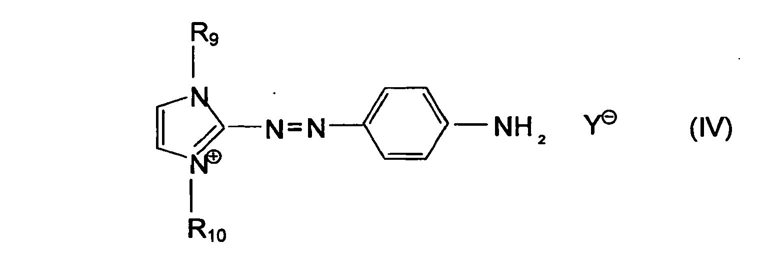 wherein R₉, and R₁₀ are methyl and Y is chloride.

5. Composition according to any of the preceding claims **characterized in that** it comprises component a at a concentration of 0.01 to 5% by weight calculated to total composition excluding oxidizing agent.

6. Composition according to any of the preceding claims **characterized in that** it comprises component b at a concentration of 0.1 to 7.5% by weight calculated to total composition excluding oxidizing agent.

7. Composition according to any of the preceding claims **characterized in that** it comprises component c at a concentration of 0.01 to 2.5% by weight calculated to total composition excluding oxidizing agent.

8. Composition according to any of the preceding claims **characterized in that** it comprises components b and c at a weight ratio of (cationic dyestuff : anionic dyestuff) 3:1 to 1:10, preferably 2:1 to 1:7.

9. Composition according to any of the preceding claims **characterised in that** it comprises additionally at least one coupling substance selected from resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2.6-diaminopyridine, 1.3-diaminobenzene, 1-amino-3-(2'-hydroxyethylamino)benzene, 1-amino-3-[bis(2'-hydroxy-ethyl) amino]benzene, α-naphthol, 4.6-dichlororesorcinol, 1.3-diaminotoluene, 1-hydroxy naphthalene, 4-hydroxy-1.2-methy-lenedioxy benzene, 1.5-dihydroxy naphthalene, 1.6-dihydroxy naphthalene, 1.7-dihydroxy naphthalene, 2.7-dihydroxy naphthalene, 1-hydroxy-2-methyl naphthalene, 4-hydroxy-1.2-methyldioxy benzene, 2.4-diamino-3-chlorophenol and/or 1-methoxy-2-amino-4-(2'-hydroxyethyl amino)benzene or the water-soluble salts thereof.

10. Composition according to any of the preceding claims **characterized in that** it comprises organosiloxane polymer according to formula 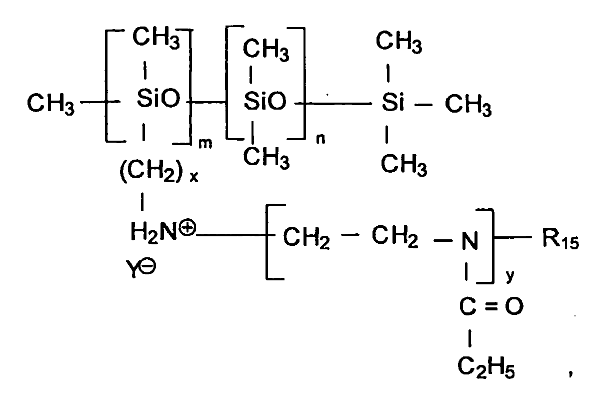 wherein m and n each are numbers from 20 to 10,000, x is a number between 1 and 5, and y is a number from 5 to 30, R₁₅ is a C₁-C₁₂-alkyl or aryl group, in particular a methyl, ethyl or benzyl group, and Y- is an anion.

11. Composition according to any of the preceding claims **characterized in that** it comprises additionally at least one ceramide type compound according to the formula 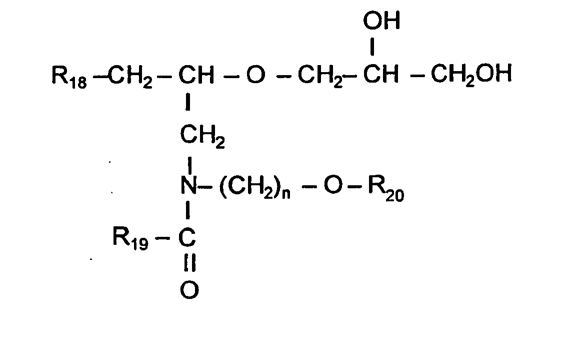 where R₁₈ and R₁₉ are independent from each other alkyl-or. alkenyl group with 10 to 22 carbon atoms, R₂₀ is methyl, ethyl, n-propyl or isopropyl group
and n is a number between 1 to 6, preferably 2 or 3.

12. Composition according to any of the preceding caharactersed in that it comprises of at least one ubiquinone of the formula 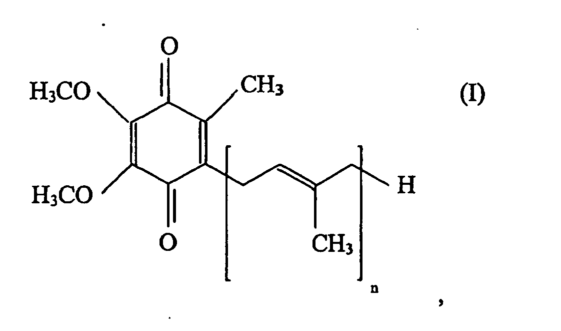 wherein n is a number from 1 to 10.

13. Composition according to any of the preceding claims **characterized in that** it comprises additional cationic and/or neutral HC dyes.

14. Composition according to any of the preceding claims **characterized in that** it comprises additionally potassium or sodium iodide and/or dihydroxyacetone for as catalysts.

15. Use of compositions according to any of the preceding claims for durably coloring keratin fibers, especially hair.

## Patentansprüche

1. Zusammensetzung zum Färben von Keratinfasern, speziell menschlichen Haaren, **dadurch gekennzeichnet, dass** sie
a- mindestens einen Oxidations- Farbentwickler, ausgewählt aus Pyrazol oder dessen wasserlösliche Salze,
b- mindestens einen sauren Direktfarbstoff, ausgewählt aus den Verbindungen Acid Red 52, DC Violet 2, DC Red 33, DC Orange 4, und DC Yellow 10, und
c- mindestens einen kationischen Direktfarbstoff, ausgewählt aus Verbindungen, dargestellt durch die allgemeine Formeln III, IV and V 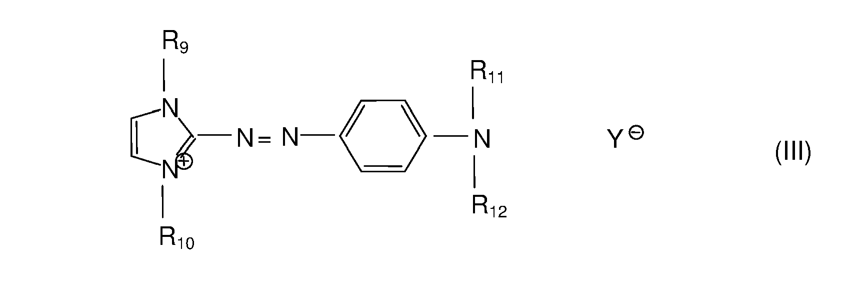 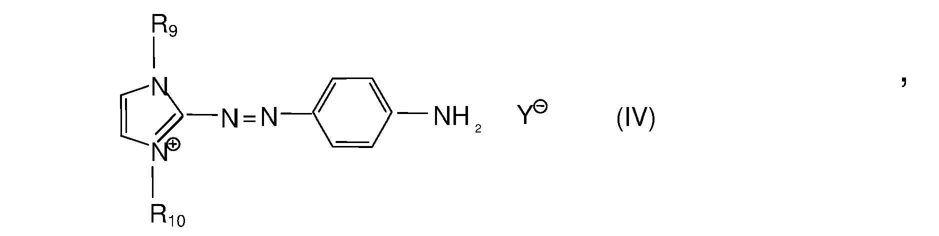 und 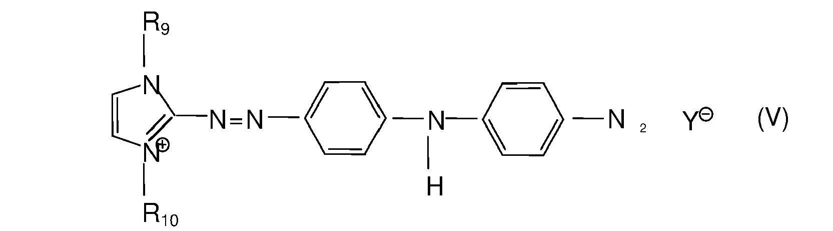 enthält,
worin R₉, R₁₀, R₁₁ und R₁₂ für Wasserstoff, eine CH₃- oder C₂H₅- Gruppe steht, und Y ein Anion wie Chlorid, Bromid oder Methosulfat ist.

2. Zusammensetzung zum Färben von Keratinfasern, speziell menschlichen Haaren, **dadurch gekennzeichnet, dass** sie
a mindestens einen Oxidations- Farbentwickler, ausgewählt aus den Verbindungen
1-Hydroxyethyl-4,5-diaminopyrazole, 3,4-Diamino-5-hydroxypyrazole, 3,5-Diaminopyrazole, 3,5-Diamino pyrazol-1-carboxamide, 3-Amino-5-hydroxypyrazole, 1-Phenyl-2-methylpyrazole, 1-Phenyl-3-methylpyrazole-5-one, 3,5-Dimethylpyrazole, 3,5-Dimethylpyrazole-1-methanol, 1-Methyl-4,5-diaminopyrazole, 1-Methylethyl-4,5-diaminopyrazole, 1-Phenylmethyl-4,5-diaminopyrazole, 1-Methyl-4,5-diaminopyrazole, 1-(4-Methylphenyl)methyl-4,5-diaminopyrazole, 1-Methyl-3-phenyl-4,5-diaminopyrazole und die wasserlöslichen Salze,
b mindestens einen sauren Direktfarbstoff, ausgewählt aus den Verbindungen Acid Red 52, DC Violet 2, DC Red 33, DC Orange 4, und DC Yellow 10, und
c mindestens einen kationischen Direktfarbstoff, ausgewählt aus den Verbindungen, dargestellt mit der allgemeinen Formel III, IV und V 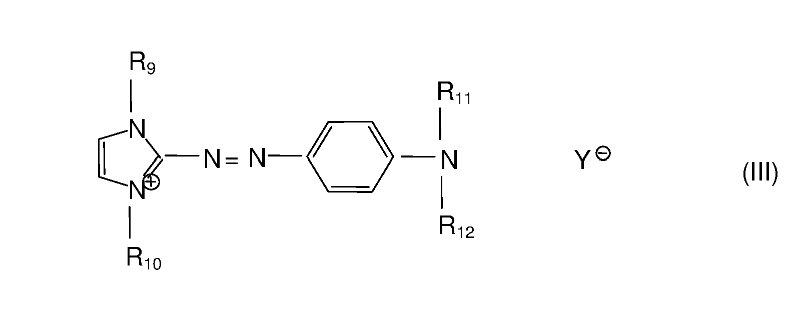 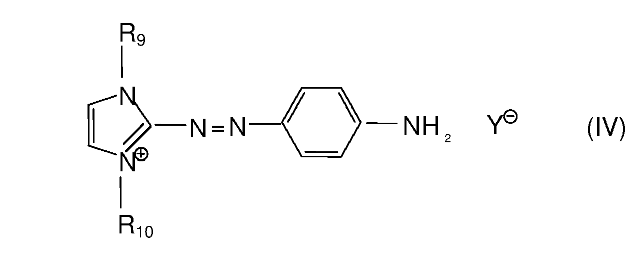 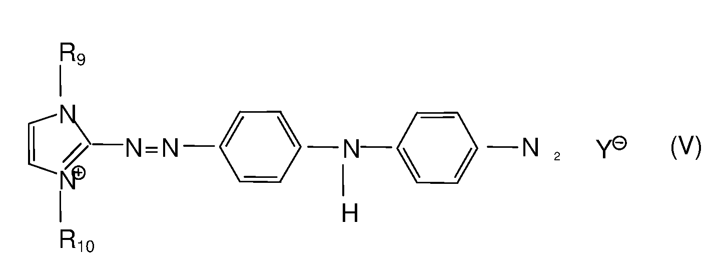 enthält,
worin R₉, R₁₀, R₁₁ und R₁₂ für Wasserstoff, eine CH₃- oder C₂H₅- Gruppe steht, und Y ein Anion wie Chlorid, Bromid oder Methosulfat ist.

3. Zusammensetzung nach einem der vorhergehenden Ansprüche, **dadurch gekennzeichnet, dass** sie als Komponente c den kationischen Farbstoff entsprechend der Formel III enthält 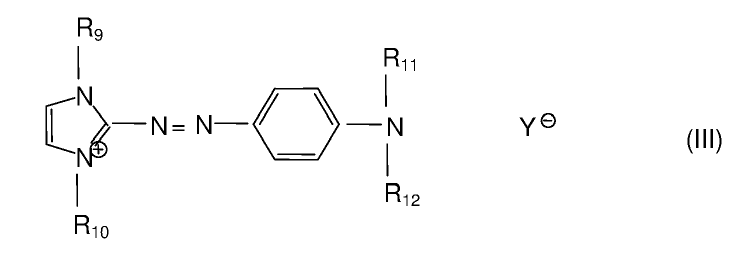 worin R₉, R₁₀, R₁₁ und R₁₂ Methyl ist und Y Chlorid ist.

4. Zusammensetzung nach den Ansprüchen 1 bis 3, **dadurch gekennzeichnet, dass** sie als Komponente c den kationischen Farbstoff entsprechend der Formel IV enthält 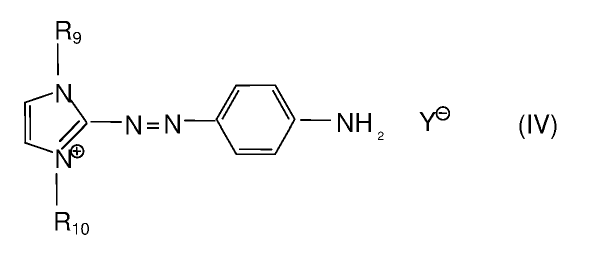 worin R₉, und R₁₀ Methyl ist und Y Chloride ist.

5. Zusammensetzung nach einem der vorhergehenden Ansprüche, **dadurch gekennzeichnet, dass** sie die Komponente a in einer Menge von 0,01 bis 5 Gew.-% enthält, berechnet auf die Gesamtzusammensetzung, ausgenommen Oxidationsmittel.

6. Zusammensetzung nach einem der vorhergehenden Ansprüche, **dadurch gekennzeichnet, dass** sie die Komponente b in einer Menge von 0,1 bis 7,5 Gew.-% enthält, berechnet auf die Gesamtzusammensetzung, ausgenommen Oxidationsmittel.

7. Zusammensetzung nach einem der vorhergehenden Ansprüche, **dadurch gekennzeichnet, dass** sie Komponente c in einer Menge von 0,01 bis 2,5 Gew.- % enthält, berechnet auf die Gesamtzusammensetzung, ausgenommen Oxidationsmittel.

8. Zusammensetzung nach einem der vorhergehenden Ansprüche, **dadurch gekennzeichnet, dass** sie die Komponenten b und c in einem Gewichtsverhältnis von (kationischenFarbstoff : anionischen Farbstoff) 3:1 bis 1:10, vorzugsweise 2:1 bis 1:7 enthält.

9. Zusammensetzung nach einem der vorhergehenden Ansprüche, **dadurch gekennzeichnet, dass** sie zusätzlich mindestens eine Kupplersubstanz enthält, ausgewählt aus Resorcinol, 2-Methylresorcinol, 4-Chlororesorcinol, 2-Amino-4-chlorophenol, 5-Amino-4-methoxy-2-methylphenol, 3-Amino-phenol, 1-Methyl-2-hydroxy-4-aminobenzene, 3-N,N-Dimethylaminophenol, 2.6-Dihydroxy-3.5-dimethoxypyridine, 5-Amino-3-methylphenol, 6-Amino-3-methylphenol, 3-Amino-2-methylamino-6-methoxypyridine, 2-Amino-3-hydroxy-pyridine, 2-Dimethyl-amino-5-aminopyridine, 2.6-Diaminopyridine, 1.3-Diaminobenzene, 1-Amino-3-(2'-hy-droxyethylamino)benzene, 1-Amino-3-[bis(2'-hydroxy-ethyl) amino]benzene, α-Naphthol, 4.6-Dichlororesorcinol, 1.3-Diaminotoluene, 1-Hydroxy naphthalene, 4-Hydroxy-1.2-methy-lenedioxy benzene, 1.5-Dihydroxy naphthalene, 1.6-Dihydroxy naphthalene, 1.7-Dihydroxy naphthalene, 2.7-Dihydroxy naphthalene, 1-Hydroxy-2-methyl naphthalene, 4-Hydroxy-1.2-methyldioxy benzene, 2.4-Diamino-3-chlorophenol und/oder 1-Methoxy-2-amino-4-(2'-hydroxyethyl amino)benzene oder deren wasserlösliche Salze.

10. Zusammensetzung nach einem der vorhergehenden Ansprüche, **dadurch gekennzeichnet, dass** sie Organosiloxane- Polymer enthält, entsprechend der Formel 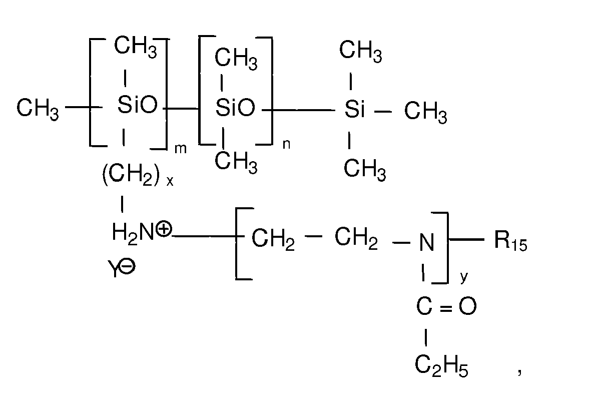 worin m und n jeweils Zahlen von 20 bis 10,000 sind, x eine Zahl zwischen 1 und 5 ist, und y eine Zahl von 5 bis 30 ist, R₁₅ eine C₁-C₁₂-Alkyl- oder Aryl-Gruppe ist, speziell eine Methyl-, Ethyl- oder Benzyl- Gruppe, und Y⁻ ein Anion ist.

11. Zusammensetzung nach einem der vorhergehenden Ansprüche, **dadurch gekennzeichnet, dass** sie zusätzlich mindestens eine Ceramid- Typen Verbindung nach der allgemeinen Formel 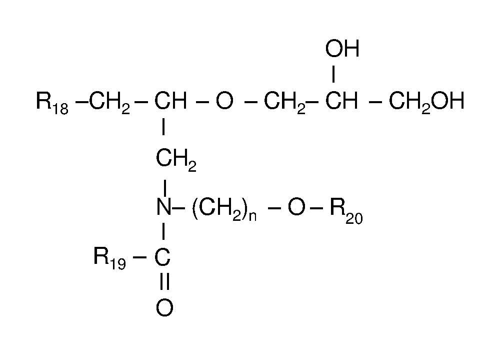 enthält,
worin R₁₈ und R₁₉ unabhängig voneinander Alkyl- oder. Alkenyl- Gruppen mit 10 bis 22 Kohlenstoffatomen sind, R₂₀ Methyl-, Ethyl-, n-Propyl- oder Isopropyl- Gruppen sind und n eine Zahl zwischen 1 bis 6, vorzugsweise 2 oder 3 ist.

12. Zusammensetzung nach einem der vorhergehenden Ansprüche, **dadurch gekennzeichnet, dass** sie mindestens ein Ubichinon der Formel 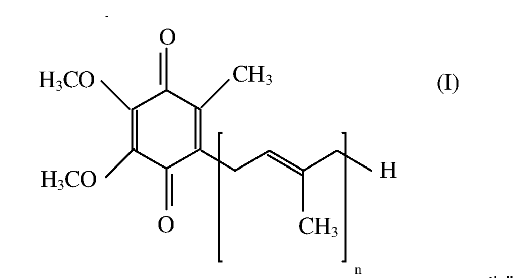 enthält worin n eine Zahl von 1 bis 10 ist.

13. Zusammensetzung nach einem der vorhergehenden Ansprüche, **dadurch gekennzeichnet, dass** sie zusätzlich kationische und/oder neutrale HC Farben enthält.

14. Zusammensetzung nach einem der vorhergehenden Ansprüche, **dadurch gekennzeichnet, dass** sie zusätzlich Kalium- oder Natriumjodid und/oder Dihydroxyaceton als Katalysatoren enthält.

15. Verwendung von Zusammensetzungen nach einem der vorhergehenden Ansprüche zur beständigen Färbung von Keratinfasern, speziell Haaren.

## Revendications

1. Compositions pour la teinture de fibres de kératine, notamment de cheveux humains, **caractérisée en ce qu'**elle comprend
a- au moins un précurseur de colorant par oxydation choisi parmi du pyrazole ou ses sels hydrosolubles,
b- au moins un colorant direct acide choisi parmi les composés Acid Red 52, DC Violet 2, DC Red 33, DC Orange 4, et DC Yellow 10, et
c- au moins un colorant direct cationique choisi parmi les composés présentés par les formules générales III, IV et VI 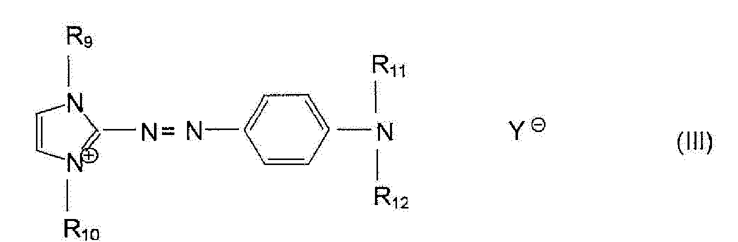 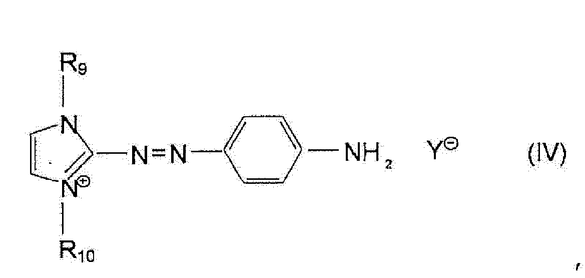 et 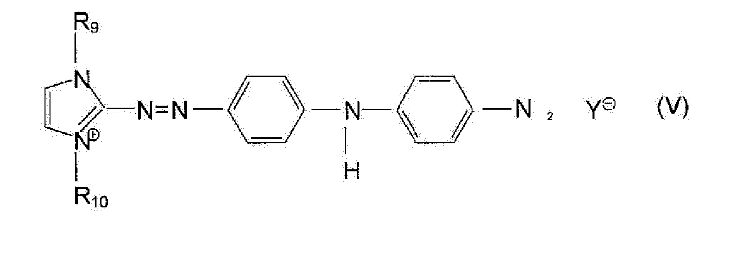 dans lesquelles R₉, R₁₀, R₁₁ et R₁₂ représentent un atome d'hydrogène, un groupe CH₃- ou C₂H₅-, et Y est un anion tel qu'un chlorure, un bromure, un méthosulfate.

2. Composition pour la teinture de fibres de kératine, notamment de cheveux humains, **caractérisée en ce qu'**elle comprend
a au moins un précurseur de colorant par oxydation choisi parmi les composés 1-hydroxyéthyl-4,5-diaminopyrazole, 3,4-diamino-5-hydroxypyrazole, 3,5-diaminopyrazole; 3,5-diaminopyrazol-1-carboxamide, 3-amino-5-hydroxypyrazole, 1-phényl-2-méthylpyrazole, 1-phényl-3-méthylpyrazole-5-one, 3,5-diméthylpyrazole, 3,5-diméthylpyrazole-1-méthanol, 1-méthyl-4,5-diaminopyrazole, 1-méthyléthyl-4,5-diaminopyrazole, 1-phénylméthyl-4,5-diaminopyrazole, 1-méthyl-4,5-diaminopyrazole, 1-(4-méthylphényl)méthyl-4,5-diaminopyrazole, 1-méthyl-3-phényl-4,5-diaminopyrazole et les sels hydrosolubles,
b au moins un colorant direct acide choisi parmi les composés Acid Red 52, DC Violet 2, DC Red 33, DC Orange 4, et DC Yellow 10, et
c au moins un colorant direct cationique choisi parmi les composés présentés par les formules générales III, IV et VI 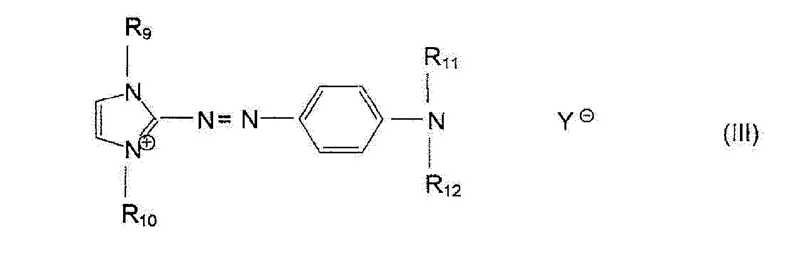 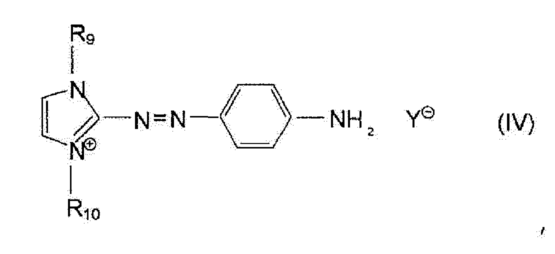 et 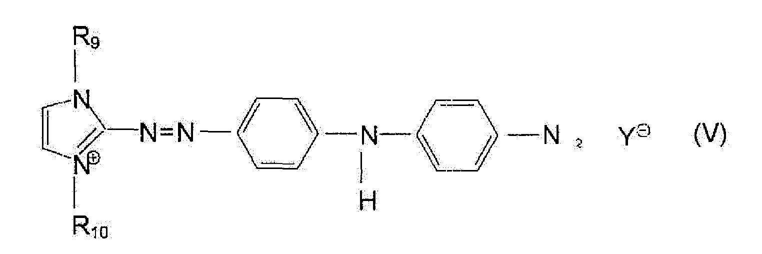 dans lesquelles R₉, R₁₀, R₁₁ et R₁₂ représentent un atome d'hydrogène, un groupe CH₃- ou C₂H₅-, et Y est un anion tel qu'un chlorure, un bromure, un méthosulfate.

3. Composition selon l'une quelconque des revendications précédentes, **caractérisée en ce qu'**elle comprend en tant que composant c, la matière colorante cationique selon la formule III 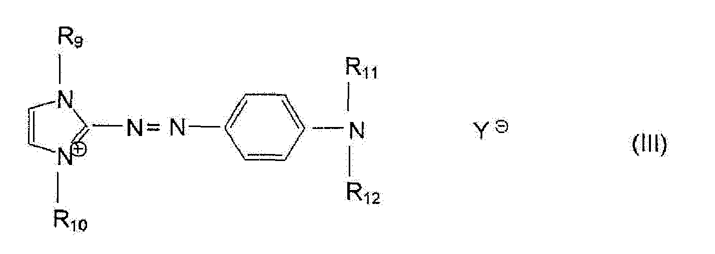 dans laquelle R₉, R₁₀, R₁₁ et R₁₂ sont un groupe méthyle et Y est un chlorure.

4. Composition selon les revendications 1 à 3, **caractérisée en ce qu'**elle comprend en tant que composant c, la matière colorante cationique selon la formule IV 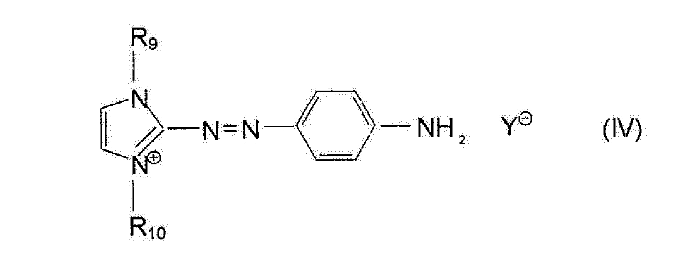 dans laquelle R₉ et R₁₀ sont un groupe méthyle et Y est un chlorure.

5. Composition selon l'une quelconque des revendications précédentes, **caractérisée en ce qu'**elle comprend le composant a à une concentration de 0,01 à 5 % en poids calculée par rapport à la composition totale à l'exclusion d'un agent d'oxydation.

6. Composition selon l'une quelconque des revendications précédentes, **caractérisée en ce qu'**elle comprend le composant b à une concentration de 0,1 à 7,5 % en poids calculée par rapport à la composition totale à l'exclusion d'un agent d'oxydation.

7. Composition selon l'une quelconque des revendications précédentes, **caractérisée en ce qu'**elle comprend le composant c à une concentration de 0,01 à 2,5 % en poids calculée par rapport à la composition totale à l'exclusion d'un agent d'oxydation.

8. Composition selon l'une quelconque des revendications précédentes, **caractérisée en ce qu'**elle comprend les composants b et c dans un rapport en poids
(matière colorante cationique/matière colorante anionique) de 3:1 à 1:10, de préférence de 2:1 à 1:7.

9. Composition selon l'une quelconque des revendications précédentes, **caractérisée en ce qu'**elle comprend en outre au moins une substance de couplage choisie parmi du résorcinol, du 2-méthylrésorcinol, du 4-chlororésorcinol, du 2-amino-4-chlorophénol, du 5-amino-4-méthoxy-2-méthylphénol, du 3-aminophénol, du 1-méthyl-2-hydroxy-4-aminobenzène, du 3-N,N-diméthylaminophénol, de la 2,6-dihydroxy-3,5-diméthoxypyridine, du 5-amino-3-méthyl-phénol, du 6-amino-3-méthylphénol, de la 3-amino-2-méthyl-amino-6-méthoxypyridine, de la 2-amino-3-hydroxypyridine, de la 2-diméthylamino-5-aminopyridine, de la 2,6-diamine-pyridine, du 1,3-diaminobenzène, du 1-amino-3-(2'-hydroxy-éthylamino)benzène, du 1-amino-3-[bis(2'-hydroxyéthyl)-amino]benzène, de l'α-naphtol, du 4,6-dichlororésorcinol, du 1,3-diaminotoluène, du 1-hydroxynaphtalène, du 4-hydroxy-1,2-méthylènedioxybenzène, du 1,5-dihydroxynaphtalène, du 1,6-dihydroxynaphtalène, du 1,7-dihydroxynaphtalène, du 2,7-dihydroxynaphtalène, du 1-hydroxy-2-méthylnaphtalène, du 4-hydroxy-1,2-méthyldioxybenzène, du 2,4-diamino-3-chlorophénol et/ou du 1-méthoxy-2-amino-4-(2'-hydroxyéthylamino)benzène ou leurs sels, hydrosolubles.

10. Composition selon l'une quelconque des revendications précédentes, **caractérisée en ce qu'**elle comprend un polyorganosiloxane selon la formule 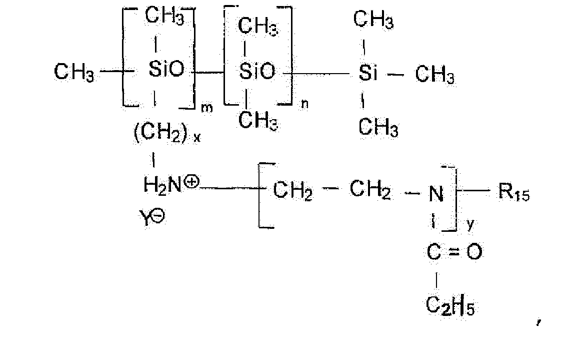 dans laquelle m et n sont chacun des nombres allant de 20 à 10 000, x est un nombre compris entre 1 et 5, et y est un nombre allant de 5 à 30, R₁₅ est un groupe alkyle en C₁-C₁₂ ou aryle, en particulier un groupe méthyle, éthyle ou benzyle, et Y⁻ est un anion.

11. Composition selon l'une quelconque des revendications précédentes, **caractérisée en ce qu'**elle comprend en outre au moins un composé de type céramide selon la formule 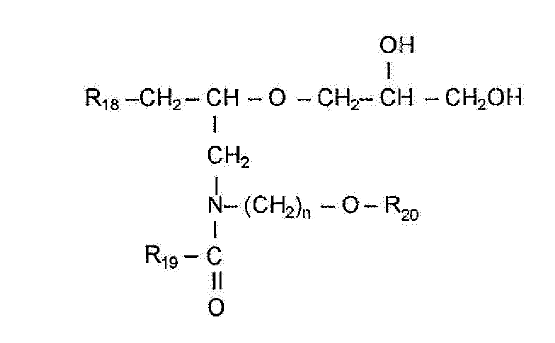 dans laquelle R₁₈ et R₁₉ sont, indépendamment l'un de l'autre, un groupe alkyle ou alcényle avec 10 à 22 atomes de carbone, R₂₀ est un groupe méthyle, éthyle, n-propyle ou isopropyle et n est un nombre compris entre 1 et 6, de préférence 2 ou 3.

12. Composition selon l'une quelconque des revendications précédentes, **caractérisée en ce qu'**elle comprend au moins une ubiquinone de formule 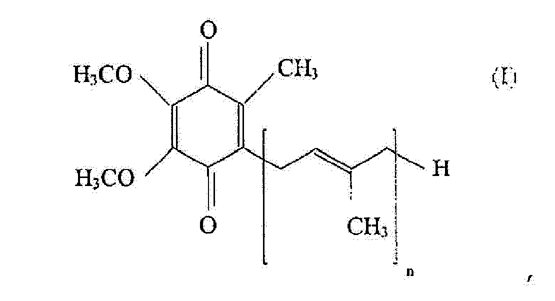 dans laquelle n est un nombre allant de 1 à 10.

13. Composition selon l'une quelconque des revendications précédentes, **caractérisée en ce qu'**elle comprend des colorantes HC cationiques et/ou neutres supplémentaires.

14. Composition selon l'une quelconque des revendications précédentes, **caractérisée en ce qu'**elle comprend en outre de l'iodure de potassium ou de sodium et/ou de la dihydroxyacétone pour servir de catalyseurs.

15. Utilisation de compositions selon l'une quelconque des revendications précédentes pour colorer durablement des fibres de kératine, notamment des cheveux.
